# EUROPEAN PATENT APPLICATION

(11) **EP 1 985 313 A1**
(43) Date of publication of application: **29.10.2008**
(21) Application number: 07714382.4
(22) Date of filing: 16.02.2007
(51) Int. Cl.: A61K 51/00

(54) **POSITRON TOMOGRAPHY METHOD AND POSITRON-EMITTING COMPOUND TO BE USED THEREIN**

(30) Priority: 17.02.2006 JP 2006041545
(71) Applicant: Nard Institute, Ltd., Amagasaki-shi Hyogo 6600805 (JP); National University Corporation Chiba University, Chiba-shi Chiba, 2638522 (JP)
(72) Inventor: MATSUO, Masaaki, Amagasaki-shi, Hyogo 660-0805 (JP); KITASHOJI, Takeru, Amagasaki-shi, Hyogo 660-0805 (JP); KOBASHI, Tatsuhiro, Amagasaki-shi, Hyogo 660-0805 (JP); TAKAHAGI, Makoto, Amagasaki-shi, Hyogo 660-0805 (JP); TSUKADA, Hideo, Hamamatsu-shi, Shizuoka 435-8558 (JP); NISHIYAMA, Shingo, Hamamatsu-shi, Shizuoka 435-8558 (JP); HASHIMOTO, Kenji, Chiba-shi, Chiba 260-8670 (JP)
(74) Representative: Weber, Thomas
(86) International application number: PCT/JP2007/052852
(87) International publication number: WO 2007/094455

(57) **Abstract**

It is an objective of the present invention to provide a positron tomography method applicable to the diagnosis of specific brain diseases and to provide a positron-emitting compound for use in the method. The present invention method relates to a positron tomography method for measuring a distribution and concentration of a positron-emitting source, and is **characterized in** using the compound (I) as the positron-emitting source. 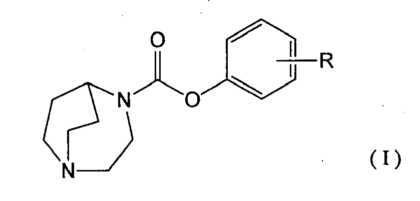 [wherein, R represents a C₁₋₆ alkyl group or a halogen atom group, having positron-emitting capability]

## Description

### TECHNICAL FIELD

The present invention relates to a positron tomography method and a positron-emitting compound for use therein.

### BACKGROUND ART

A positron, i.e. positive electron, emitted from a positron-emitting source disappears to produce a pair of γ rays, i.e. annihilation radiation. In positron tomography, the pair of γ rays is converted into electrons, and the electrons are counted in real time by a detector. Devices for use in such positron tomography are disclosed, for example, in Japanese publications of unexamined application Nos. 6-273529 and 8-211154. The positron tomography enables the measurement of the distribution and accumulated concentration of the positron-emitting source and thus is used to diagnose some diseases.

Specifically, for example, ¹⁸F-labeled fluorodeoxyglucose administered into the body is more taken up by cancer cells, because cancer cells divide more rapidly than normal cells and need more glucose. When this state is imaged using a positron tomograph, the distribution and accumulated concentration of the fluorodeoxyglucose can be measured so that the presence or absence and the size of the cancer lesion can be determined.

Further, positron tomography can reveal that administered ¹⁸F-fluorodeoxyglucose is more concentrated in the brain, since brain cells also consume more energy than other cells. When brain cells are partially damaged for some reason, the uptake of glucose into the damaged part may decrease. Therefore, the presence or absence of brain dysfunction can be diagnosed by positron tomography.

Consequently, positron tomography is applicable to the diagnosis of diseases and thus greatly expected to undergo further development in the future.

It is known that there are at least 12 subtypes of nicotine receptors α2 to 10 and β2 to 4. Among them, α7 is suspected to be related to Alzheimer disease. Specifically, it is reported in Kenji Hashimoto and Masaomi Iyo, "The Amyloid Cascade Hypothesis of Alzheimer Disease and α7 Nicotine Receptor", Journal for Japanese Society of Neuropsychopharmacology, Vol. 22, pp. 49-53 (2002) that an immuno-histochemical method using the dead brain of an Alzheimer disease patient revealed a reduction in the density of α7 nicotine receptor in the frontal cortex of the patient. It is also known that β amyloid protein that are considered as having an important role in Alzheimer disease bind to α7 nicotine receptor with a very high affinity, but their affinity for other subtypes α4β2 is about 5,000 times weaker than that for α7 nicotine receptor. It is also considered that α7 nicotine receptor may be related to disturbance of attention and information processing disorder in schizophrenia.

Ligands for α7 nicotine receptor deservingly include nicotine. However, the affinity between nicotine and α7 nicotine receptor is not so high, and nicotine has low specificity to α7 nicotine receptor and can bind to other receptors. Thus, various selective ligands for α7 nicotine receptor have been examined.

For example, published Japanese translation of PCT international publication No. 2002-540208 describes ligands for α7 nicotine receptor and discloses that such compounds are useful for the treatment of Alzheimer disease and other diseases. Various other compounds are also known, which are considered to selectively bind to α7 nicotine receptor.

### DISCLOSURE OF THE INVENTION

As described above, there have been some examples in which positron tomography is used for the examination of brain function. However, positron tomography has not yet been used to diagnose more specific brain diseases. It is therefore an objective of the present invention to provide a positron tomography method applicable to the diagnosis of specific brain diseases and to provide a positron-emitting compound useful for such a method.

The inventors had the idea that if positron tomography is carried out using any α7 nicotine receptor-selective ligand in which a substituent having positron emission capability is introduced, the distribution and concentration of α7 nicotine receptor in the brain might be determined in relation to various diseases. However, it has been found that some compounds actually bind to parts other than α7 nicotine receptor and do not allow accurate measurement, even though the compounds are considered to be capable of selectively binding to α7 nicotine receptor. It has also been found that some ligands for α7 nicotine receptor are not applicable to positron tomography of the brain, because the compounds cannot pass through the blood-brain barrier when the compounds are administered by injection. Therefore, the inventors have made investigations on compounds applicable to positron tomography. As a result, it has been found that the compound (I) described below is very suitable for positron tomography, because the blood-brain barrier is highly permeable to the compound (I) and selective binding of the compound (I) to α7 nicotine receptor is particularly excellent, so that the present invention has been completed.

The positron tomography method of the present invention for measuring a distribution and concentration of a positron-emitting source is characterized in that a compound (I) is used as the positron-emitting source. 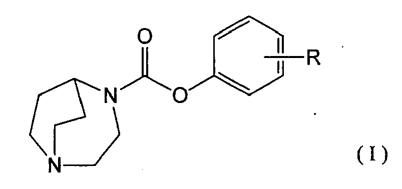 [wherein, R represents a C₁₋₆ alkyl group or a halogen atom group, having positron-emitting capability]

The method may include the steps of using a specific positron-emitting source and detecting the decay thereof with measuring equipment, but does not include medical practice or action on a human body with equipment, and thus falls within industrially applicable inventions under the Japanese Patent Law.

The term "C₁₋₆ alkyl group" refers to a straight or branched chain aliphatic hydrocarbon having 1 to 6 carbon atoms. For example, the C₁₋₆ alkyl group may be methyl, ethyl, propyl, isopropyl, butyl, isobutyl, tert-butyl, pentyl, isoamyl or hexyl. A C₁₋₄ alkyl group is preferred, a C₁₋₂ alkyl group is more preferred, and a methyl group is even more preferred.

The "halogen atom group" may be a fluorine atom, a chlorine atom, a bromine atom or an iodine atom. Among them, a chlorine, bromine or iodine atom is preferred, and a bromine or iodine atom is more preferred, and a bromine atom is even more preferred.

The present invention also relates to a positron-emitting source compound (I): 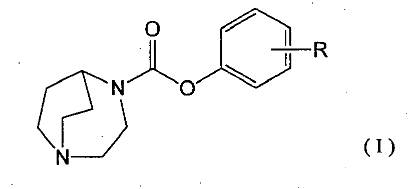 [wherein, R represents a C₁₋₆ alkyl group or a halogen atom group, having positron-emitting capability]

In the positron-emitting source compound (I), R is preferably ¹¹CH₃ or ⁷⁶Br, and the substituent R is preferably at position 4. Particularly preferred are the compounds (Ia) and (Ib):

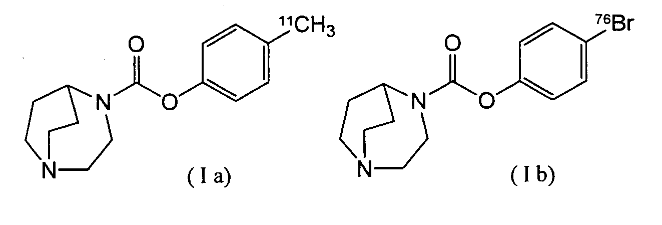

### BRIEF DESCRIPTION OF THE DRAWINGS

Fig. 1 is a schematic diagram of a system for synthesizing the positron-emitting compound of the present invention.
Fig. 2 is a graph showing the time course of the distribution of compound (Ia) in each part of the brain after the administration of compound (Ia) by intravenous injection.
Fig. 3 is a graph showing the time course of the distribution of compound (Ib) in each part of the brain after the administration of compound (Ib) by intravenous injection.

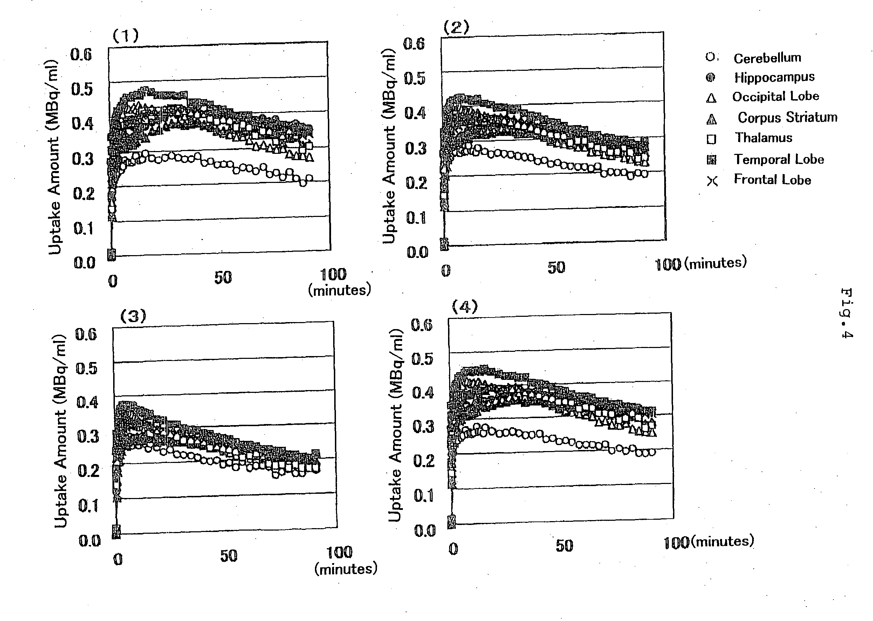

Fig. 4 shows graphs of the time course of the distribution of compound (Ia) in each part of the brain after the administration of a selective agonist or the like followed by the administration of compound (Ia) by intravenous injection, in which Part (1) shows the result of a control experiment, Part (2) shows the result of an experiment in which SSR180711A, an α7 nicotine receptor-selective agonist, was administered in a dose of 1 mg/kg weight, Part (3) shows the result of an experiment in which SSR180711A was administered in a dose of 5 mg/kg weight, and Part (4) shows the result of an experiment in which A85380, an α4β2 nicotine receptor-selective agonist, was administered.

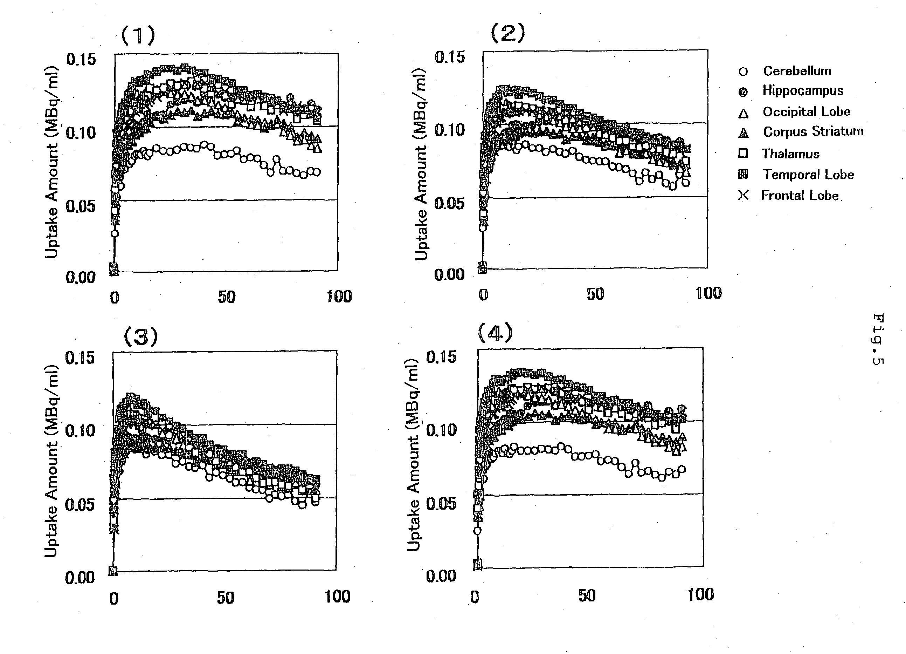

Fig. 5 shows graphs of the time course of the distribution of compound (Ib) in each part of the brain after the administration of a selective agonist or the like followed by the administration of compound (Ib) by intravenous injection, in which Part (1) shows the result of a control experiment, Part (2) shows the result of an experiment in which SSR180711A, an α7 nicotine receptor-selective agonist, was administered in a dose of 1 mg/kg weight, Part (3) shows the result of an experiment in which SSR180711A was administered in a dose of 5 mg/kg weight, and Part (4) shows the result of an experiment in which A85380, an α4β2 nicotine receptor-selective agonist, was administered.

### BEST MODE FOR CARRYING OUT THE INVENTION

The positron tomography method of the present invention includes measuring the distribution and concentration of a positron-emitting source, and is characterized in that a compound (I) is used as the positron-emitting source. 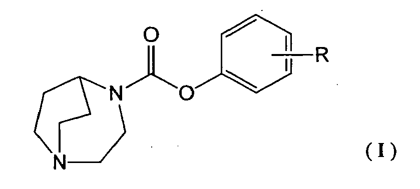 [wherein, R represents a C₁₋₆ alkyl group or a halogen atom group, having positron-emitting capability]

The method of the present invention relates to positron tomography in which the distribution and concentration of the positron-emitting source is measured. A positron-emitting element gradually decays with emitting a positron, i.e. positive electron. While moving to a few millimeters, the positron loses energy by collision and couples with a negative electron to disappear. In this process, a pair of γ rays is emitted in opposite directions at 180° to each other. In positron tomography, the γ rays are converted into electrons to be measured in real time so that the location where the positron disappears can be detected and that the distribution and concentration of the positron-emitting source can be measured.

In the method of the present invention, the compound (I) is used as a positron-emitting source. The compound (I) is an excellent positron-emitting source, because the blood-brain barrier is highly permeable to the compound (I), the compound (I) can selectively bind to α7 nicotine receptor, and is less likely to bind to other portions in the brain.

In the compound (I), [¹¹C]C₁₋₆ alkyl group, ¹⁸F, ⁷⁶Br and ¹²³I can be exemplified as the R group. The compound having ¹¹CH₃ as the R group is an excellent positron-emitting source, because the blood-brain barrier is highly permeable to the compound and the compound has high selectivity to α7 nicotine receptor. The compound having ⁷⁶Br as the R group also has high selectivity and is excellent in terms of convenience, because ⁷⁶Br has a relatively long half-life of 12 hours and is easy to handle.

The substituent R is preferably at position 4, while the substituent may be at any position. The examples described later demonstrate high permeability of the blood-brain barrier to such a compound and highly selective binding of such a compound to α7 nicotine receptor.

In view of the half-life of the positron-emitting element, a method for producing the compound (I) preferably includes introducing the R group including the positron-emitting element in the final synthesis step. An example of such a method is illustrated below.

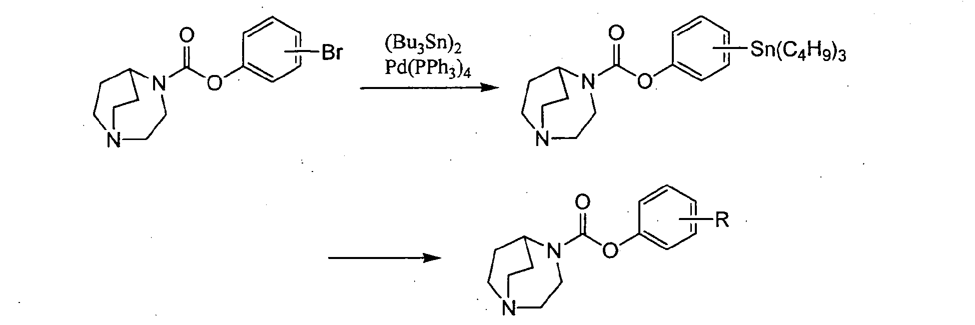

In this scheme, the reagent to introduce the R group depends on the type of the R group. For example, when the R group is a [¹¹C]C₁₋₆ alkyl group, an iodide compound of [¹¹C]C₁₋₆ alkyl or the like may be used as the reagent to introduce the R group. When the R group is ¹⁸F, ⁷⁶Br or ¹²³I, ¹⁸F⁻, ⁷⁶Br⁻ or ¹²³I⁻ may be used to introduce the R group.

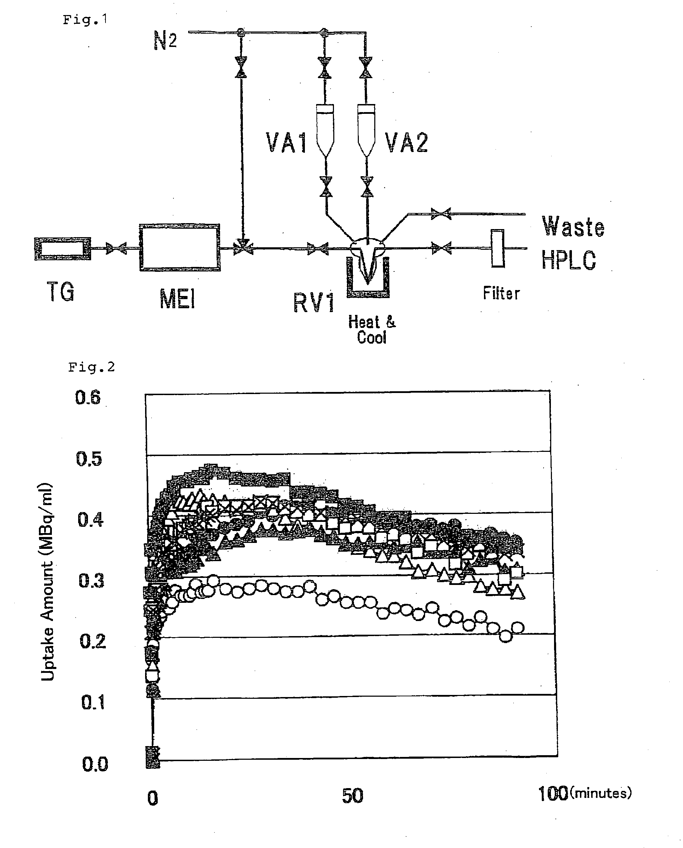

Among these reagents, an iodide compound of [¹¹C]C₁₋₆ alkyl is preferably prepared using a cyclotron or the like immediately before the introduction of the [¹¹C]C₁₋₆ alkyl group and then a reaction for the introduction of R group should be promptly carried out to produce the compound having [¹¹C]C₁₋₆ alkyl group as the R group, because the half-life of ¹¹C is 20 minutes. It is preferred that after the preparation, the iodide should be immediately subjected to the R group introduction reaction to form the compound having the [¹¹C]C₁₋₆ alkyl group as the R group. When [¹¹C]C₁₋₆ alkyl group is introduced, therefore, a synthesis system as shown in Fig. 1 should be used.

Specifically, when the compound having the R group derived from ¹¹CH₃I is produced, a positive electron beam accelerated by a cyclotron is irradiated to a target (TG in Fig. 1) that is used depending on the desired positron-emitting element, so that ¹¹CO₂ is obtained, which contains a positron-emitting element. The ¹¹CO₂ is reduced with LiAlH₄ or the like, for example, in Microlabo (MEI) and then is allowed to react with hydroiodic acid to give ¹¹CH₃I. The ¹¹CH₃I is then allowed to react with a precursor introduced from a spitch (VA1) in a reactor (RV1), so that the compound (I) can be synthesized.

When the compound having, as the R group, a [¹¹C]C₁₋₆ alkyl group other than that from ¹¹CH₃I is produced, a Grignard reagent whose carbon number is one less than that of the desired [¹¹C]C₁₋₆ alkyl group, for example, CH₃MgBr for the introduction of [¹¹C]CH₃CH₂, is added at the stage where the ¹¹CO₂ is obtained. Thereafter, a similar process may be carried out to introduce [¹¹C]C₁₋₆ alkyl group.

On the other hand, the halogen ion having positron emission capability has a relatively long half-life. For example, ⁷⁶Br⁻ has a half-life of 12 hours. Therefore, the halogen ion having positron emission capability may be purchased as a commercially available product, while the halogen ion may be prepared as needed, as described above.

The resulting positron-emitting compound is preferably formulated into an injection product, since the compound (I) has a short half-life and thus should be quickly allowed to reach the brain. Such an injection product may be prepared by conventional methods. For example, a solution or suspension in a saline may be prepared. The concentration of the injection product is preferably such that measurement can be sufficiently carried out with no side effect, while the concentration depends on the permeability of the blood-brain barrier, the radioactivity of the compound and so on. For example, when the R group is ¹¹CH₃, the concentration of the injection product may be from about 0.1 to about 0.5 µg/mL, and when the R group is ⁷⁶Br, the concentration may be from about 4 to about 20 µg/mL.

The positron tomography method of the present invention may be carried out using any known positron tomograph. Specifically, after the compound (I) is administered in the form of an injection product to a subject, the biodistribution and concentration of the compound (I) is measured with a known positron tomograph. A specific disease can be diagnosed based on the information on the relationship between the disease and α7 nicotine receptor. For example, when the compound (I) is less distributed in the frontal cortex than normal so that a reduction in the density of α7 nicotine receptor is recognized, Alzheimer disease may be diagnosed.

According to conventional techniques, the dose of the compound (I) can be appropriately controlled depending on the symptom, condition, sex, age or the like of the subject. For example, the dose of the compound having ¹¹CH₃ as the R group may be from about 1.5 to about 10 ng/kg weight, and the dose of the compound having ⁷⁶Br as the R group may be from about 50 to about 350 ng/kg weight. The injection product may be administered in an amount of 1 to 10 mL.

The method of the present invention described above may be useful to diagnose α7 nicotine receptor-related diseases. As demonstrated in the examples described later, the positron-emitting compound (I) according to the invention can pass through the blood-brain barrier and reach the brain upon intravenous administration and selectively bind to α7 nicotine receptor, so that the distribution and concentration of α7 nicotine receptor in the brain can be measured.

The present invention is more specifically described by the examples below. It should be understood that the scope of the present invention is not restricted by the examples described below in any way, and any appropriate modifications can be made without departing the gist of the invention described above or below, and such modifications all fall within the scope of the invention.

### EXAMPLES

### Production Example 1-1: Synthesis of 4-tributyltinphenyl 1,4-diazabicyclo[3.2.2]nonane-4-carboxylate

In dry toluene (30 mL), 4-bromophenyl 2,5-diazabicyclo[3.2.2] nonane-2-carboxylate (1.0 g, 0.003 mol) was dissolved, and tetrakistriphenylphosphine palladium (0.18 g, 0.00015 mol) was added thereto. Bistributyltin (4.45 g, 0.0077 mol) was then added dropwise, and the mixture was stirred for 16 hours under heating and reflux. After the reaction was completed, the reaction mixture was filtered. The solvent was removed from the filtrate by distillation under reduced pressure. The resultant product was then purified by column chromatography with NH silica gel and a mobile phase of n-hexane/ethyl acetate=3/2, to give the title compound (0.7 g, yield: 43%).

### Production Example 1-2: Synthesis of ¹¹CH₃I

Electrons were accelerated to 18 MeV with a cyclotron (HM-18 manufactured by Sumitomo Heavy Industries, Ltd.) and applied to a pure nitrogen gas-filled target at a current of 20 µA for about 60 minutes so that ¹¹CO₂ was obtained by a ¹⁴N (p, α) ¹¹C nuclear reaction. The ¹¹CO₂ was introduced for 5 minutes into a 0.1 M LiAlH₄ tetrahydrofuran solution (500 µL) cooled to -10°C. Tetrahydrofuran was then removed by distillation with N₂ gas, and then hydriodic acid (0. 5 mL) was added to the residue. The resulting CH₃I was purified by distillation.

### Production Example 1-3: Synthesis of 4-[¹¹C]methyl-phenyl 1,4-diazabicyclo[3.2.2]nonane-4-carboxylate as compound (Ia)

Tris(dibenzylideneacetone)dipalladium (4.5 mg) and tri-O-tolylphosphine (6.2 mg) were dissolved in dimethylformamide (0.3 mL) and the mixture was slightly heated. The ¹¹CH₃I obtained in Production Example 1-2 was added to the resulting solution. When the radioactivity reached equilibrium, the reactor was hermetically sealed, and the palladium complex was allowed to react with the ¹¹CH₃I at room temperature for one minute. Separately, the tributyltin compound (4 mg) obtained in Production Example 1-1, cupper chloride (CuCl, 1.0 mg) and potassium carbonate (1.4 mg) were dissolved in dimethylformamide (0.3 mL). The solution was added to the ¹¹CH₃I solution, and the mixture was subjected to a methylation reaction by heating at 70°C for 5 minutes. The resulting reaction mixture was purified by high performance liquid chromatography under the conditions below, to give the title compound (Ia) (7 µg, yield: 30 to 40%). The resulting compound (Ia) was dissolved in a saline (5 to 10 mL), and the solution was filtered through a 0.22 µm sterilization filter to give an injection product.

Conditions for the High Performance Liquid Chromatography
Column: Megapak SIC C18-10, 7. 6 x 250 mm (manufactured by JASCO Corporation)
Eluent: acetonitrile/30 mM ammonium acetate/acetic acid=600/400/2
Flow rate: 6 mL/minute
Detection wavelength: 220 nm

The resulting compound (Ia) had the characteristics shown in Table 1.

**[Table 1]**

| | |
|---|---|
| Production Amount | 900 MBq |
| Radiochemical Purity | 99.9 % |
| Specific Radioactivity | 33.2 GBq/µmol |

### Production Example 2: Synthesis of 4-[⁷⁶Br]bromophenyl 1,4-diazabicyclo[3.2.2]nonane-4-carboxylate as compound (Ib)

The 4-tributyltinphenyl 1,4-diazabicyclo[3.2.2]nonane-4-carboxylate obtained in Production Example 1-1 (1 mg, 1.9 µmol) was dissolved in a 1% ethanol solution of acetic acid (250 µL). Separately, a commercially available [⁷⁶Br]HBr solution (15 to 20 mL, manufactured by S.H.I. Examination & Inspection, Ltd.) was concentrated to about 200 µL at 75°C under a nitrogen gas stream. The solution of the compound of Production Example 1-1 and a 200 mM ethanol solution of chloramine T (25 µL) were added to the [⁷⁶Br]HBr solution (about 200 µL), and the mixture was allowed to react at 75°C for 30 minutes. After the reaction was completed, the product was purified by high performance liquid chromatography (with Megapak SIC C18-10, 7.6 x 250 mm, manufactured by JASCO Corporation, at a flow rate of 6 mL/minute and a wavelength of 220 nm), using a mixed solvent of acetonitrile/ (1000 mL of 30 mM ammonium acetate and 4 mL of acetic acid)=1/1 as an eluent. The resulting compound (Ib) had the characteristics shown in Table 2.

**[Table 2]**

| | |
|---|---|
| Production Amount | 24.8 MBq |
| Radiochemical Purity | 100 s% |
| Specific Radioactivity | 7.5 GBq/µmol |

The resulting compound (Ib) (1 µg) was dissolved in a saline (3.3 mL), to form an injection product.

### Test Example 1: Test for determining the amount of uptake of positron-emitting source compound

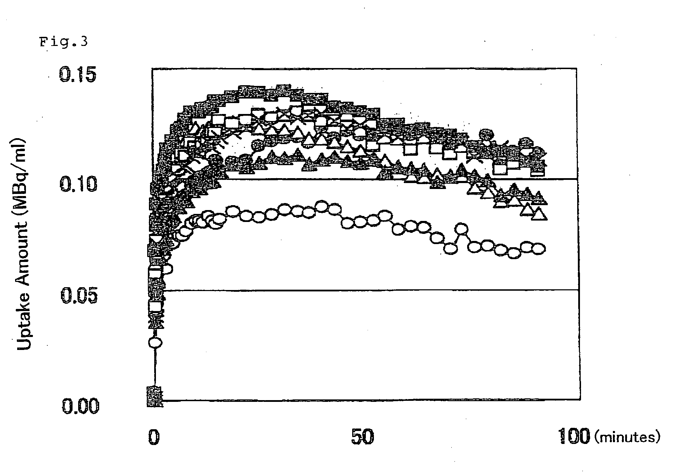

A rhesus monkey (male, 5.5 kg in weight) was fasted overnight prior to the assay date. The monkey was seated on a monkey chair, and its head was fixed in a gantry of an animal PET camera (SHR-7700 manufactured by Hamamatsu Photonics K.K.) by a head positioner. Transmission measurement was performed for 30 minutes for respiration compensation. The injection products prepared in Production Examples 1-3 and 2 were then intravenously administered in doses of 77 ng/kg weight in terms of the amount of compound (Ia) and 230 ng/kg weight in terms of the amount of compound (Ib), respectively, and dynamic measurement was performed for 180 minutes. After the obtained images were reconstituted, a region of interest (ROI) was set in each part of the brain, and kinetics of radioactivity was determined in each region. The results on compounds (Ia) and (Ib) are shown in Figs. 2 and 3, respectively.

As is evident from the result in Fig. 2, the compound having ¹¹CH₃ as the R group, i.e. Compound (Ia), has excellent permeability to the blood-brain barrier and the compound is taken up in parenchymal cells of the brain very well. As far as the integrated images 40 to 60 minutes after the administration were examined, the distribution of the concentrated compound is high in hippocampus, corpus striatum, thalamus, cingulate gyrus and cerebral cortex, but low in cerebellum. The results well agree with the distribution of α7 nicotine receptor in the brain. The kinetics of compound (Ia) in the brain were relatively fast and showed peak values about 10 minutes after the administration in most parts, except for the hippocampus where the kinetics was relatively slow and showed a peak value 30 minutes after the administration.

As is evident from Fig. 3, the kinetics of the compound having ⁷⁶Br as the R group, i.e. compound (Ib), was generally slow in the brain as compared with the case of compound (Ia) and showed peak values 60 minutes after the administration in hippocampus and 30 to 40 minutes after the administration in other parts, respectively. The amount of uptake of compound (Ib) is not as large as that of compound (Ia). However, compound (Ib) has excellent permeability to the blood-brain barrier, and the distribution of compound (Ib) in the brain well agrees with that of α7 nicotine receptor.

### Test Example 2: Test for selectivity to α7 nicotine receptor

In order to examine the selectivity of each compound to α7 nicotine receptor, SSR180711A which is an α7 nicotine receptor-selective agonist, or A85380 which is an α4β2 nicotine receptor-selective agonist, was administered in advance, and then the amount of uptake of compound (Ia) or (Ib) was measured.

Specifically, transmission measurement was first performed under similar condition to Test Example 1, and then SSR180711A (1 or 5 mg/kg weight) or A85380 (5 mg/kg weight) was administered by intravenous injection. After 30 minutes, compound (Ia) or (Ib) was administered by intravenous injection, and dynamic measurement was performed for 91 minutes. After the obtained images were reconstituted, a region of interest (ROI) was set in each part of the brain, and kinetics of radioactivity was determined in each region. The results on compounds (Ia) and (Ib) are shown in Figs. 4 and 5, respectively. For comparison, the result of Test Example 1 in which nothing was administered in advance is shown as Part (1) in each drawing.

As is evident from the results in Figs. 4 and 5, a comparison between Part (1) as control and Part (4) in which α4β2 nicotine receptor-selective agonist was administered in advance indicates that there was almost no effect on the uptake of compound (Ia) or (Ib) into each part of the brain. On the other hand, when α7 nicotine receptor-selective agonist was administered in advance, i.e. in Parts (2) and (3), the uptake of compound (Ia) or (Ib) into each part of the brain was reduced to the level in the cerebellum where the content of the α7 receptor was very low, depending on the dose of the selective agonist. The results have demonstrated that compounds (Ia) and (Ib) according to the present invention exhibit selective binding to α7 nicotine receptor and thus the method of the present invention enables the diagnosis of α7 nicotine receptor-related diseases.

### INDUSTRIAL APPLICABILITY

The compound (I) of the present invention can quickly reach the brain upon administration by injection, regardless of the existence of the blood-brain barrier, and can truly and selectively bind to α7 nicotine receptor. Therefore, the use of the compound (I) as a positron-emitting source in positron tomography allows accurate measurement of the distribution and concentration of α7 nicotine receptor in the brain. Consequently, the method of the present invention is useful for the diagnosis of α7 nicotine receptor-related diseases such as Alzheimer disease, schizophrenia, cognition disorder, hyperkinetic syndrome, anxiety neurosis, depression, epilepsy, analgesia, Tourette syndrome, Parkinson disease and Huntington chorea.

## Claims

1. A positron tomography method for measuring a distribution and concentration of a positron-emitting source, **characterized in that** a compound (I) is used as the positron-emitting source. 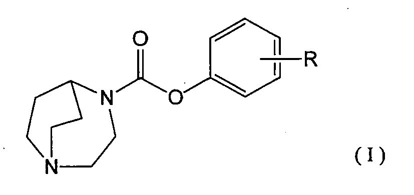 [wherein, R represents a C₁₋₆ alkyl group or a halogen atom group, having positron-emitting capability]

2. The positron tomography method according to claim 1, wherein R is ¹¹CH₃ in the compound (I) used as the positron-emitting source.

3. The positron tomography method according to claim 1, wherein R is ⁷⁶Br in the compound (I) used as the positron-emitting source.

4. The positron tomography method according to any one of claims 1 to 3, wherein the substituent R is at position 4 in the compound (I) used as the positron-emitting source.

5. A positron-emitting source compound (I) represented by the formula: 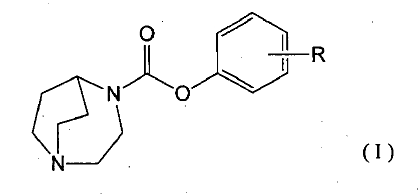 [wherein, R represents a C₁₋₆ alkyl group or a halogen atom group, having positron-emitting capability]

6. A positron-emitting source compound (Ia) represented by the formula:

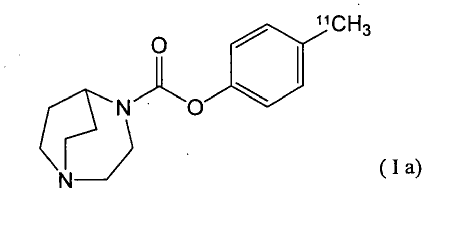

7. A positron-emitting source compound (Ib) represented by the formula:
